# EUROPÄISCHE PATENTSCHRIFT

(11) **EP 0 816 339 B1**
(45) Veröffentlichungstag und Bekanntmachung des Hinweises auf die Patenterteilung: **16.01.2002**
(21) Anmeldenummer: 97105910.0
(22) Anmeldetag: 10.04.1997
(51) Int. Cl.: C07D 209/52

(54) **Verfahren zur Herstellung von (+-)2-Azabicyclo(2.2.1)hept-5-en-3-on**
Process for the preparation of (+-)2-Azabicyclo[2.2.1]hept-5-en-3-one
Procédé pour la préparation de (+-)2-Azabicyclo[2.2.1]hept-5-en-3-one

(30) Priorität: 25.06.1996 DE 19625323
(43) Veröffentlichungstag der Anmeldung: 07.01.1998
(73) Patentinhaber: Degussa AG, 40474 Düsseldorf (DE)
(72) Erfinder: Romanowski, Frank, Dr., 2140 Borgerhout (BE); Vroman, Kurt, 2200 Morkhoven (BE); Vanheertum, Rudolf, Dr., 63796 Kahl (DE)

(56) Entgegenhaltungen:
- EP-A- 0 508 352
- EP-A- 0 533 048
- NOBUYA KATAGIRI: "Hetero Diels-Alder Reaction of Benzenesulfonyl Cyanide with cyclopentadiene using Chiral Lewis Acids" CHEMICAL & PHARMACEUTICAL BULLETIN, Bd. 44, Nr. 4, April 1996, JAPAN, Seiten 850-851, XP002045993
- GARETH J. GRIFFITHS ET AL.: "Diels alder Reaction of Methanesulfonyl cyanide with cyclopentadiene. Industrial Synthesis of 2-Azabicyclo[2.2.1]hept-5-en-3-one" THE JOURNAL OF ORGANIC CHEMISTRY, Bd. 58, Nr. 22, 1993, COLUMBUS OHIO, Seiten 6129-6131, XP002045994
- SUSAN DALUGE ET AL.: "Synthesis of Carbocyclic Aminonucleosides" THE JOURNAL OF ORGANIC CHEMISTRY, Bd. 43, Nr. 12, 1978, COLUMBUS OHIO, Seiten 2311-2320, XP002045995
- J.C.JAGT ET AL.: "Diels alder cycloadditions of Sulfonylcyanides with Cyclopentadiene. Synthesis of 2-Azabicyclo[2.2.1]hepta-2,5-dienes" THE JOURNAL OF ORGANIC CHEMISTRY, Bd. 39, Nr. 4, 1974, COLUMBUS OHIO, Seiten 564-566, XP002045996
- CHEMICAL ABSTRACTS, vol. 124, no. 23, 3.Juni 1996 Columbus, Ohio, US; abstract no. 316997a, Seite 1201; Spalte 2; XP002045997 & JP 08 027 110 A (KURARAY CO.) 30.Januar 1996
- CHEMICAL ABSTRACTS, vol. 121, no. 9, 29.August 1994 Columbus, Ohio, US; abstract no. 108525f, Seite 1046; Spalte 2; XP002045998 & JP 05 331 139 A (KURARAY CO.) 14.Dezember 1993
- CHEMICAL ABSTRACTS, vol. 121, no. 9, 29.August 1994 Columbus, Ohio, US; abstract no. 108526g, Seite 1046; Spalte 2; XP002045999 & JP 05 331 140 A (KURARAY CO,) 14.Dezember 1993

## Beschreibung

Die vorliegende Erfindung bietet ein vereinfachtes Verfahren zur Herstellung von ± 2-Azabicyclo[2.2.1]hept-5-en-3-on aus Cyclopentadien und Sulfocyaniden als Ausgangsmaterialien, wobei das Zielprodukt in hoher Reinheit und hoher Ausbeute erhalten wird.

### Anwendungsgebiet

± 2-Azabicyclo[2.2.1]hept-5-en-3-on ist Ausgangsmaterial zur Herstellung von karbozyklischen Nukleosidanalogen,die wegen ihrer antiviralen und chemotherapeutischen Eigenschaften als Heilmittel in der Medizin von Interesse sind.

### Stand der Technik

Nach einer in der Literatur beschriebenen Methode setzt man festes isoliertes p-Toluolsulfonylcyanid in einem großen Überschuß mit Cyclopentadien um, das gleichzeitig als Lösungsmittel fungiert. Vince et al., J. Org. Chem. 43 (1978), 2311; J.C. Jagt et al., J. Org. Chem. 39 (1974), 564.Es wird dann die sehr instabile Zwischenverbindung 3-Tosyl-2-azabicyclo[2.2.1]hepta-2,5-dien, dem Diels-Alder-Produkt aus Cyclopentadien und p-Toluolsulfocyanid, isoliert durch Aufkonzentration der erhaltenen Lösung. Der Feststoff wird anschließend mit Eisessig umgesetzt und danach durch Zugabe von Wasser hydrolysiert, wobei die Tosylgruppe abgespalten wird.

Bei der zweiten Methode werden Cyclopentadien und Chlorosulfonylisocyanat unter Ringschluß aneinander angelagert und die Chlorsulfonylgruppe unter Verwendung von Natriumsulfit abgespalten (J.R. Malpass et al., J. Chem. Soc., Perkin I, (1977), 874.

Aus der EP-A 0508 352 ist ein Verfahren bekannt, bei dem man 1,3-Cyclopentadien mit Methansulfonylcyanid in einem organischen Lösungsmittel umsetzt.
Die EP-A 0533 048 betrifft ein Kreislaufverfahren zur Herstellung von Lactamen, bei dem dieselbe Reaktion in Gegenwart von organischen Lösungsmitteln und Wasser stattfindet. Der pH-Wert der Reaktionsmischung muß während der Reaktion durch Zusatz von Lauge konstant gehalten werden. Wie man jedoch bei G.H. Griffiths und F.E. Previdoli (J. Org. Chem. 58 (1993) 6129-6131) lesen kann, erhält man in wäßriger Lösung mit p-Toluolsulfinat das gewünschte Produkt nur in einer niedrigen Ausbeute und unzureichender Reinheit.

In zwei japanischen Offenlegungsschriften (Hei 5-331139 und Hei 5-331140, 14.12.1993)wird eine Methode beschrieben, bei der die immer als Festsubstanz eingesetzten Sulfonylcyanide in einem organischen Lösungsmittel oder Wasser aufgeschlämmt werden und Cyclopentadien zu dieser Suspension zugegeben wird.
Nachteil dieses Verfahrens ist die Zwischenisolierung der nicht als sehr stabil bekannten Sulfonylcyanide, die sich unter erheblicher Wärmeentwicklung zersetzen können.

Aufgabe der Erfindung ist die Bereitstellung eines verbesserten Verfahrens zur Herstellung von (±)2-Azabicyclo[2.2.1]hept-5-en-3-on(RAN).

Gegenstand der Erfindung ist ein Verfahren zur Herstellung von 2-Azabicyclo[2.2.1]hept-5-en-3-on durch Umsetzung von 1.3-Cyclopentadien mit einem substituierten Sulfonylcyanid in Gegenwart von Wasser, und Abwesenheit eines organischen Lösungsmittels, das dadurch gekennzeichnet ist, daß man
a) ein Sulfinat der allgemeinen Formel

   RSO₂X (I),

   in der bedeuten
   - R:: Ethyl, Propyl, Benzyl, 4-Methylbenzyl, p-Tolyl 4-Nitrobenzyl, 2-Nitrobenzyl, bevorzugt Ethyl, Propyl, besonders bevorzugt 4-Methylbenzyl, Benzyl
   - X:: ein Alkalikation Na oder K, H in Wasser als einzigem Lösungsmittel mit Chlorcyan zu einem Sulfonylcyanid der allgemeinen Formel

   RSO₂CN (II)

   umsetzt,
b) dieses ohne Zwischenisolierung anschließend mit 1 - 2 Moläq. 1.3-Cyclopentadien bei einem pH-Wert von 2,5 - 7 und einer Temperatur von -10 bis +4°C reagieren läßt,
c) den bei der Reaktion entstehenden Niederschlag des Nebenprodukts (Sulfinat) abfiltriert,
d) den pH-Wert des Filtrats mit einer wässrigen Alkalihydroxidlösung auf einen Wert zwischen 7 und 8,5 einstellt und
e) das gewünschte Produkt daraus extrahiert und durch Abtrennen des Lösungsmittels gewinnt.

Insbesondere geeignet sind Sulfinate, die in Wasser löslich sind.
Verbindungen dieses Typs stellt man beispielsweise aus den entsprechenden Sulfonylchloriden und z. B. Natriumsulfit in situ her.

Die sich anschließende Umsetzung mit Chlorcyan, das entweder flüssig oder gasförmig in die wässrige Lösung eingeleitet wird, erfolgt im allgemeinen bei Temperaturen von -10 °C bis +15 °C.
Die sich daraus ergebende HCl-Bildung führt zu einem pH-Wert der Lösung, der im sauren Bereich liegt.
Die Verfahrensführung erfordert jedoch im Gegensatz zum Stand der Technik keine Regulierung dieses Werts durch ständige Zugabe von z. B. Natronlauge.
Das Chlorcyan wird in einer solchen Menge zugesetzt, daß es im allgemeinen bis zur Zugabe des Cyclopentadien abreagiert ist.

Das sich bildende Sulfonylcyanid der allgemeinen Formel

RSO₂CN (II),

in der R die obengenannte Bedeutung besitzt, fällt im allgemeinen in Form eines Feststoffs an, so daß sich eine Suspension bildet.

Die Wassermenge liegt im allgemeinen bei der 10 bis 150-fachen Molmenge, bezogen auf das Sulfonylcyanid, insbesondere bei dem 20 bis 80-fachen.

Dieser Suspension, in der das Sulfonylcyanid gegebenenfalls teilweise gelöst vorliegt, setzt man Cyclopentadien, bevorzugt frisch destilliert, in einem Molverhältnis von 1 bis 2,0 zu 1 Mol Sulfonylcyanid, insbesondere von bis zu 1,5 zu. Die Umsetzung erfolgt dann bei einer Temperatur von -10 bis +40 °C, insbesondere -10 bis 30 °C.

Während dieser Reaktion liegt der pH-Wert der Reaktionsmischung insbesondere zwischen 2,5 und 7. Man rührt bis zu vollständigen Umsetzung im allgemeinen 2 bis 5 h

Eine ständige Nachregulierung des pH-Werts ist nicht notwendig.

Die anfallende Reaktionsmischung kann man auf verschiedenen Wegen aufarbeiten.
In einer bevorzugten Ausführungsform trennt man die als festes Nebenprodukt anfallende Sulfinsäure durch geeignete Filtrierschritte ab, bringt das Filtrat anschließend auf einen pH-Wert von 7 bis 8,5, insbesondere ca. 8, und gewinnt das gewünschte Produkt nach dem Austreiben eines gegebenenfalls vorhandenen Cyclopentadienüberschusses, durch Extraktion mit einem organischen inerten Lösungsmittel.

Als Extraktionsmittel kommen mit Wasser nicht mischbare organische Lösungsmittel wie chlorierte Kohlenwasserstoffe, Ketone, Ether und dergleiche in Betracht. Bevorzugte Lösungsmittel sind Methylenchlorid, Methyltert.butylether, Chloroform, Methylisobutylketon, Methylisopropylketon, Nitromethan, Nitroethan, 1- und 2-Nitropropan. Durch Eindampfung der organischen Lösung erhält man das Endprodukt in reiner Form.
Die Filtration der z. B. als Nebenprodukt anfallenden p-Toluolsulfinsäure geschieht bevorzugt unter Stickstoffatmosphäre aufgrund der Zersetzungseigenschaften. Das Nebenprodukt wird durch Auflösen in C1- bis C5-Alkoholen, vorzugsweise Methanol, Ethanol, n-Propanol, iso-Propanol, in eine sichere Transportform überführt. Obwohl das Nebenprodukt ab 30 °C zur Zersetzung neigt, sind die Lösungen bis 80 °C stabil.

Das Nebenprodukt kann auch in Wasser supendiert und in Alkali gelöst werden. Durch Einsatz von Natronlauge wird zum Beispiel eine wässrige Lösung des Natrium-p-toluolsulfinats erhalten. Diese Arbeitsweise hat den Vorteil, daß die Sulfinatlösung zurückgeführt und mit Chlorcyan erneut zum Sulfonylcyanid umgesetzt werden kann.

Eine weitere Möglichkeit den in isolierter Form instabilen Nebenproduktniederschlag nicht als Feststoff zu isolieren, besteht in der Neutralisation des Reaktionsgemisches auf pH-Werte zwischen 5 und 10, vorzugsweise 6,5 und 8,5. Dadurch geht der Niederschlag vollständig in Lösung. Das Zielprodukt fällt nach der Extraktion und Aufkonzentrierung im Gegensatz zum Stand der Technik in reiner Form kristallin an.

Die Qualität des Zielproduktes ist weiterhin zu steigern, indem man das von dem Nebenprodukt abgetrennte Filtrat mit Aktivkohle reinigt. Die wässrige Lösung des Zielproduktes wird im allgemeinen schon vorher durch pH-Einstellung auf 6,5 bis 8,5 stabilisiert, anschließend gegebenenfalls gereinigt, extrahiert und aufkonzentriert.

In einer bevorzugten Variante wird das Sulfonylcyanid in situ durch Umsetzung des entsprechenden Sulfonylchlorids mit Natriumsulfit gewonnen (JP-OS 5600 753) und anschließend mit Chlorcyan umgesetzt.

### Beispiel 1

In 300 ml Wasser wurden 60,2 g (0,338 Mol) Natrium-p-toluolsulfinat vorgelegt, auf -3 ° C abgekühlt und 20 ml ClCN in 10 Min. zugegeben. Man ließ 1 h bei -3 ° C rühren und gab 37 ml (0,47 Mol) frisch hergestelltes Cyclopentadien hinzu. Man erwärmte auf 18 ° C und ließ 3 h bei dieser Temperatur rühren.
Anschließend wurde der Niederschlag abfiltriert und noch im Druckfilter mit Methanol gelöst.

Das Filtrat wurde auf pH 8 gebracht, 1 h mit Aktivkohle behandelt und mit CH₂Cl₂ extrahiert. Nach Aufkonzentrieren erhielt man 29,5 g (0,27 Mol) hellbeiges RAN entsprechend 80 % Ausbeute.

### Beispiel 2

In 350 ml Wasser wurden 49 g (0,39 Mol) Natriumsulfit aufgelöst und die Lösung auf 105 ° C erwärmt. In mehreren Portionen wurden in 75 Min. 68 g (0,357 Mol) p-Toluolsulfonylchlorid zugefügt und der Ansatz 3 h bei 100 ° C bis 105 ° C nachgerührt.
Der pH-Wert wurde während der Reaktion durch Zufügen von insgesamt 67 ml 30 %-iger NaOH zwischen 5 und 7 gehalten.

Das Reaktionsgemisch wurde auf -3 ° C abgekühlt und anschließend 20 ml Chlorcyan zugefügt.
Man ließ 1 h bei dieser Temperatur rühren und fügte dann 24 g (0,36 Mol) Cyclopentadien hinzu. Das Reaktionsgemisch wurde auf 15 ° C erwärmt und bei dieser Temperatur 3 h gerührt.

Das ausgefallene Nebenprodukt wurde abfiltriert und in Methanol gelöst.
Das Filtrat wurde auf pH 8,6 eingestellt und der Cyclopentadienüberschuß ausgetrieben.
Die wäßrige RAN-Lösung wurde mit Methylenchlorid extrahiert und man erhielt nach Aufkonzentrieren 23,3 g (0,21 Mol) hellbeiges 97 %-iges RAN, entsprechend 58 % Ausbeute.

### Beispiel 3

In 300 ml Wasser wurden 60,2 (0,338 Mol) Natrium-p-toluolsulfinat vorgelegt, auf -3 ° C abgekühlt und 20 ml Chlorcyan in 10 Min. zugegeben. Man ließ 1 h bei -3 ° C rühren und gab 37 ml (0,47 Mol) frisch hergestelltes Cyclopentadien hinzu. Man erwärmte auf 18 ° C und ließ bei 3 h bei dieser Temperatur rühren.
Anschließend wurde mit 40 ml 30 %-iges NaOH unter Kühlung bei 20 bis 30 ° C neutralisiert. Die leicht trübe Lösung wurde filtriert und überschüssiges Cyclopentadien ausgetrieben, die Lösung mit Methylenchlorid extrahiert. Nach Aufkonzentrieren erhält man 29,4 g (0,25 Mol) 94 %-iges gelbbraunes RAN, entsprechend 75 % Ausbeute.

### Beispiel 4

In 350 ml Wasser wurden 49 g (0,39 Mol) Na₂SO₃ aufgelöst und die Lösung auf 100 ° C erwärmt. In mehreren Portionen wurden 79,8 g (0,36 Mol) Nitrobenzosulfonylchlorid zugegeben und der Ansatz 3 h bei 100 °C nachgerührt Der pH-Wert wurde während der Zugabe des Sulfonylchlorids und der Nachreaktion durch Zufügen von insgesamt 60 ml 30 %-iger NAOH zwischen 5 und 7 gehalten.

Nachdem das Reaktionsgemisch auf -3 ° C abgekühlt war, wurden 20 ml Chlorcyan zugefügt. Man ließ 1 h bei dieser Temperatur rühren und fügte dann 38 ml (0,5 Mol) Cyclopentadien zu. Das Reaktionsgemisch wurde auf 15 ° C erwärmt und 3 h bei 15 bis 20 ° C gerührt.
Das ausgefallene Nebenprodukt wurde abfiltriert und in Methanol gelöst.
Das Filtrat wurde neutralisiert und der Cyclopentadienüberschuß ausgetrieben.
Die wäßrige Lösung wurde mit Methylenchlorid extrahiert und man erhielt nach Aufkonzentrieren 21,7 g (0,2 Mol) 94 %-iges RAN, entsprechend 60 % Ausbeute, bezogen auf das Sulfinat.

### Beispiel 5

In 350 ml Wasser wurden 49 g (0,39 Mol) Na₂SO₃ und 65,5 g (0,78 Mol) NaHCO₃ gelöst und langsam 46,3 g (0,36 Mol) Ethansulfonylchlorid zugetropft. Die Lösung wurde über Nacht gerührt und vor der Zugabe von 19,5 ml (0,39 Mol) flüssigem Chlorcyan auf. -3 ° C abgekühlt. Es wurde 1 h bei 0 ° C bis -3 ° C gerührt und nachfolgend die Lösung mit 38 ml (0,48 Mol) Cyclopentadien versetzt. Nach Erwärmen auf 15 ° C wurden 3 h gerührt.
Die leicht trübe Lösung wurde filtriert mit 20 ml 30 %-iger NaOH neutralisiert und überschüssiges Cyclopentadien ausgetrieben.
Nach Extraktion und Aufkonzentrierung erhielt man 19,5 g 95 %-iges (0,17 Mol) RAN entsprechend 59 % Ausbeute bezogen auf das Sulfinat.

### Beispiel 6

In 7 l Wasser wurden 1.600 g (8,664 Mol) Natrium-p-toluolsulfinat vorgelegt, auf -3 ° C abgekühlt und 475 ml (9,44) Chlorcyan in 10 Minuten zugegeben. Man ließ 1 h bei -3 ° C rühren und gab 750 g (11,35 Mol) Cyclopentadien hinzu. Man erwärmte auf 18 ° C und ließ 3 h bei dieser Temperatur rühren.
Anschließend wurde der Niederschlag (1,327 g) abfiltriert und das Filtrat auf pH 8,5 gebracht.

Die Aufarbeitung wurde analog zum Beispiel 1 durchgeführt. Es wurden 715,7 g RAN erhalten, entsprechend 75,5 % Ausbeute.

Von dem abfiltrierten Niederschlag wurden 144 g zuspendiert in 500 ml Wasser. Durch Zugabe von 64 g 50 %-iger Natronlauge wurde der pH-Wert auf 12,7 gebracht, und die Suspension 1 h gerührt. Nach Filtration wurden 629 g einer 19 %-igen Natrium- p-toluolsulfinatlösung (0,671 Mol) erhalten.
Die Lösung wurde auf -3 °C abgekühlt und es wurden 40 ml Chlorcyan (0,795 Mol) zugegeben. Nach 1 h Rühren bei -3 °C wurden 63 g (0,953 Mol) Cyclopentadien zugegeben. Man ließ bei 20 °C 3 h nachrühren.
Die Lösung wurde filtriert, auf pH 8,6 gebracht und analog zu Beispiel 1 aufgearbeitet. Es wurden 29 g 93,7 %iges RAN entsprechend 37,1 % Ausbeute erhalten.

## Patentansprüche

1. Verfahren zur Herstellung von 2-Azabicyclo[2.2.1]hept-5-en-3-on durch Umsetzung von 1.3-Cyclopentadien mit einem substituierten Sulfonylcyanid in Gegenwart von Wasser, und Abwesenheit eines organischen Lösungsmittels, **dadurch gekennzeichnet, daß** man
a) ein Sulfinat der allgemeinen Formel
RSO₂X (I),
in der bedeuten
R: Ethyl, Propyl, Benzyl, 4-Methylbenzyl, 4-Nitrobenzyl, 2-Nitrobenzyl, p-Tolyl
X: ein Alkalikation Na oder K, H in Wasser als einzigem Lösungsmittel mit Chlorcyan zu einem Sulfonylcyanid der allgemeinen Formel
RSO₂CN (II),
umsetzt,
b) dieses ohne Zwischenisolierung anschließend mit 1 - 2 Moläq. 1.3-Cyclopentadien bei einem pH-Wert von 2,5 - 7 und einer Temperatur von - 10 bis +40°C reagieren läßt,
c) den bei der Reaktion entstehenden Niederschlag des Nebenprodukts (Sulfinat) abfiltriert,
d) den pH-Wert des Filtrats mit einer wässrigen Alkalihydroxidlösung auf einen Wert zwischen 7 und 8,5 einstellt und
e) das gewünschte Produkt daraus durch Extraktion isoliert.

2. Verfahren gemäß Anspruch 1,
**dadurch gekennzeichnet, daß** man das Filtrat mit Aktivkohle reinigt.

3. Verfahren gemäß Anspruch 1,
**dadurch gekennzeichnet, daß** man das abgetrennte Sulfinat in einem C₁-C₅-Alkohol auflöst.

4. Verfahren gemäß Anspruch 1,
**dadurch gekennzeichnet, daß** man das abgetrennt Sulfinat in wässrigen Alkalihydroxid- oder-carbonatlösungen auflöst.

5. Verfahren gemäß Anspruch 4,
**dadurch gekennzeichnet, daß** man die erhaltenen Lösungen des Sulfinats als Ausgangsverbindungen für das Verfahren einsetzt.

6. Verfahren gemäß Anspruch 1,
**dadurch gekennzeichnet, daß** man das Sulfonylcyanid in situ durch Umsetzung des entsprechenden Sulfonylchlorids mit Natriumsulfit herstellt.

7. Verfahren gemäß Anspruch 1, **dadurch gekennzeichnet, daß** man Cyclopentadien und Sulfonylcyanid in einem Verhältnis 1 bis 1,5 zu 1 einsetzt.

## Claims

1. Process for the production of 2-azabicyclo[2.2.1]hept-5-en-3-one by reacting 1,3-cyclopentadiene with a substituted sulfonyl cyanide in the presence of water and in the absence of an organic solvent,
**characterised in that**
a) a sulfinate of the general formula
RSO₂X (I),
in which
R: means ethyl, propyl, benzyl, 4-methylbenzyl, 4-nitrobenzyl, 2-nitrobenzyl, p-tolyl
X: means an alkali metal cation Na or K, H is reacted in water as the sole solvent with cyanogen chloride to yield a sulfonyl cyanide of the general formula
RSO₂CN (II),
b) the latter is then reacted without intermediate isolation with 1-2 mol equivalents of 1,3-cyclopentadiene at a pH value of 2.5-7 and a temperature of -10 to +40°C,
c) the precipitate of the secondary product (sulfinate) arising during the reaction is filtered out,
d) the pH value of the filtrate is adjusted with an aqueous alkali metal hydroxide solution to a value between 7 and 8.5 and
e) the desired product is isolated therefrom by extraction.

2. Process according to claim 1,
**characterised in that** the filtrate is purified with activated carbon.

3. Process according to claim 1,
**characterised in that** the separated sulfinate is dissolved in a C₁-C₅ alcohol.

4. Process according to claim 1,
**characterised in that** the separated sulfinate is dissolved in aqueous alkali metal hydroxide or carbonate solutions.

5. Process according to claim 4,
**characterised in that** the sulfinate solutions obtained are used as starting compounds for the process.

6. Process according to claim 1,
**characterised in that** the sulfonyl cyanide is produced *in situ* by reacting the corresponding sulfonyl chloride with sodium sulfite.

7. Process according to claim 1,
**characterised in that** cyclopentadiene and sulfonyl cyanide are used in a ratio of 1-1.5:1.

## Revendications

1. Procédé de préparation de 2-azabicyclo[2.2.1]hept-5-én-3-one par conversion de 1.3-cyclopentadiène avec un cyanure de sulfonyle substitué en présence d'eau et en l'absence d'un solvant organique, **caractérisé en ce que** :
a) on convertit un sulfinate de formule générale :
RSO₂X (I)
dans laquelle :
R désigne un groupe éthyle, propyle, benzyle, 4-méthylbenzyle, 4-nitrobenzyle, 2-nitrobenzyle, p-tolyle, et
X désigne un cation alcalin Na ou K, H dans de l'eau comme solvant unique avec du chlorocyan, en un cyanure de sulfonyle de formule générale :
RSO₂CN (II)
b) on fait ensuite réagir celui-ci sans isolement intermédiaire avec 1-2 équivalents molaires de 1.3-cyclopentadiène à une valeur du pH de 2,5-7 et à une température de -10 à +40°C,
c) on sépare par filtration le dépôt - formé au cours de la réaction - du produit secondaire (sulfinate),
d) on règle la valeur du pH du filtrat avec une solution aqueuse d'hydroxyde alcalin à une valeur entre 7 et 8,5, et
e) on isole le produit souhaité par extraction.

2. Procédé selon la revendication 1, **caractérisé en ce qu'**on épure le filtrat avec du charbon activé.

3. Procédé selon la revendication 1, **caractérisé en ce qu'**on dissout le sulfinate séparé dans un alcool en C₁-C₅.

4. Procédé selon la revendication 1, **caractérisé en ce qu'**on dissout le sulfinate séparé dans des solutions aqueuses d'hydroxyde ou de carbonate alcalin.

5. Procédé selon la revendication 4, **caractérisé en ce qu'**on utilise les solutions obtenues du sulfinate comme composés de départ pour le procédé.

6. Procédé selon la revendication 1, **caractérisé en ce qu'**on prépare le cyanure de sulfonyle in situ par conversion du chlorure de sulfonyle correspondant avec du sulfite de sodium.

7. Procédé selon la revendication 1, **caractérisé en ce qu'**on utilise du cyclopentadiène et du cyanure de sulfonyle dans un rapport de 1 à 1,5:1.
